# EUROPÄISCHE PATENTSCHRIFT

(11) **EP 0 521 043 B1**
(45) Veröffentlichungstag und Bekanntmachung des Hinweises auf die Patenterteilung: **31.08.1994**
(21) Anmeldenummer: 91906258.8
(22) Anmeldetag: 08.03.1991
(51) Int. Cl.: G01N 27/60, G01N 15/00

(54) **VORRICHTUNG ZUR AUTOMATISIERTEN POLYELEKTROLYTBESTIMMUNG**
AUTOMATIC POLYELECTROLYTE DETERMINING DEVICE
DISPOSITIF DE DETERMINATION DE POLYELECTROLYTES AUTOMATISEE

(30) Priorität: 20.03.1990 DE 4008916
(43) Veröffentlichungstag der Anmeldung: 07.01.1993
(73) Patentinhaber: Mütek Analytic GmbH, D-82211 Herrsching (DE)
(72) Erfinder: KRAH, Robert, D-8012 Ottobrunn (DE)
(74) Vertreter: Bohnenberger, Johannes, Dr.
(86) Internationale Anmeldenummer: EP9100441
(87) Internationale Veröffentlichungsnummer: WO9114940

(56) Entgegenhaltungen:
- US-A- 3 368 145
- US-A- 3 421 855
- US-A- 4 446 435
- US-A- 4 449 101
- US-A- 4 769 608

## Beschreibung

Die Erfindung betrifft eine Vorrichtung zur automatisierten Polyelektrolytbestimmung nach dem Oberbegriff des Patentanspruches 1.

Eine solche Vorrichtung ist in dem Dokument US-A-3 368 145 beschrieben.

Aus der US-Druckschrift "12th Material ISA Analysis Instr. Symposium, Houston, Texas, 1966: Vol. 4, Seiten 181-198" ist ebenfalls eine Vorrichtung dieser Art bekannt, die allerdings nicht zur automatisierten Polyelektrolytbestimmung, sondern als rein manuell bedienbares Gerät ausgebildet ist. Es werden also bei diesem Gerät Proben für die Bestimmung des Polyelektrolytverbrauchs in einem Prozeß von Hand in das Probengefäß gegeben, der Kolben wird bewegt, so daß ein Strömungspotential entsteht, welches über die Elektroden abgegriffen und gemessen wird, wobei gleichzeitig eine Titration durchgeführt wird. Nach der an sich bekannten Messung wird der Kolben aus dem Probengefäß entnommen und die Probe wird entfernt. Abschließend werden der Kolben und das Probengefäß gereinigt, um für einen nächsten Meßvorgang bereit zu sein.

In vielen Fällen muß der Polyelektrolytverbrauch innerhalb eines chemisch-technischen Prozesses überwacht werden, umden Prozeß entsprechend zu steuern. Hierbei dreht es sich beispielsweise um die Papierherstellung, die Abwasserbeseitigung oder ähnliche Vorgänge, bei welchen z. B. Flockungsmittel eingesetzt werden. Bei diesen Prozessen mußte nun bisher ständig eine Arbeitskraft bereitgestellt werden, um die notwendigen Messungen vorzunehmen, deren Ergebnisse zur Steuerung des Prozesses benötigt wurden. Zum einen ist eine solche rein manuelle Messung arbeitsaufwendig, zum anderen können die Meßergebnisse oftmals nicht schnell genug gewonnen werden, um den Prozeß korrekt zu steuern bzw. zu regeln.

Eine Vorrichtung zur Polyelektrolytbestimmung mit zusätzlicher Spülvorrichtung ist in dem Dokument US-A-4 449 101 beschrieben.

Der Erfindung liegt die Aufgabe zugrunde, eine Vorrichtung aufzuzeigen, mit deren Hilfe eine automatisierte Polyelektrolytbestimmung in einfacher und reproduzierbarer Weise durchführbar ist.

Diese Aufgabe wird durch die im Kennzeichen des Patentanspruches 1 angegebenen Merkmale gelöst.

Ein wesentlicher Punkt der Erfindung liegt darin, daß der zunächst ausschließlich zu Meßzwecken notwendige bzw. verwendete Kolben im Zusammenspiel mit dem Ventil in der Abflußleitung sowie deren Mündung am Boden des zylindrischen Abschnittes eine Pumpwirkung sicherstellt, die ein wirksames Spülen und Säubern der Vorrichtung ermöglicht, ohne daß hierzu manuell eingegriffen werden müßte. Mit eben dieser Pumpwirkung wird weiterhin der zu untersuchende Prozeßstoff ausgestoßen, so daß eine selbsttätig gesteuerte, automatisch arbeitende Anordnung getroffen werden kann.

Es wird darauf hingewiesen, daß das Spülen eines Analysebehälters zur Messung der Leitfähigkeit eines Mediums durch eine Kolben-/Zylinderanordnung mit bewegtem Kolben aus der DE-AS 25 21 009 an sich bekannt ist. Hier wird allerdings das zu messende Medium selbst als Spülmedium verwendet und darüber hinaus nicht durch eine gesonderte Abflußöffnung abgeführt. Vielmehr wird die ganze Anordnung derart in die zu untersuchende Flüssigkeit getaucht, daß die Flüssigkeit durch den oben liegenden Ringspalt zwischen Kolben und Zylinder sowohl eingesaugt als auch ausgestoßen wird.

Zur exakten Steuerung ist es von Vorteil, wenn der Antrieb einen Positionsfühler zum Abtasten der Position des Kolbens umfaßt. Wenn der Kolben mittels eines Kurbeltriebes auf- und abbewegt wird, so eignet sich ein Drehwinkelgeber, der mit der Kurbel in Wirkverbindung steht.

Zur Säuberung der Vorrichtung ist es von besonderem Vorteil, wenn ein Ultraschallschwinger mit dem Probengefäß mechanisch gekoppelt ist, so daß während des Spülvorganges die Spülflüssigkeit über das Probengefäß in Schwingungen versetzt werden kann, so daß an den Oberflächen haftende Teilchen hauptsächlich durch eine Kavitationswirkung entfernt werden.

Vorteilhafterweise besteht hierbei der Boden des Probengefäs- ses aus Metall, durch welches die Ultraschallschwingungen verlustlos in die Flüssigkeit einkoppelbar sind, während die Einkoppelung über aus Kunststoff bestehende isolierende Flächen aufgrund deren Dämpfung unzureichend ist. Zur Impedanzanpassung wird der Boden vorzugsweise als Horn ausgebildet.

Das Probengefäß umfaßt vorzugsweise ein Außengefäß aus Metall mit einem im wesentlichen zylindrischen Innenraum und einen in diesen eingepaßten (eingeschrumpften) Isolierkörper, in den der zylindrische Abschnitt und der Einfüllabschnitt als ineinanderübergehend abgestufte und offene Bohrungen eingearbeitet sind. Der vorzugsweise aus Polytetrafluorethylen gebildete Isolierkörper weist an seinem unteren Rand eine Kerbe auf, welche durch den flächigen Boden des Außengefäßes abgeschlossen wird und somit eine Leitung bildet. Durch diese Anordnung ist eine besonders vollständige Entleerung des Probengefäßes möglich, so daß kaum Reste von Spülflüssigkeit verbleiben, welche eine nachfolgende Probe verändern könnten.

Von besonderem Vorteil ist es, wenn die Vorrichtung eine Probenentnahmeeinrichtung umfaßt, die eine Pumpe aufweist, welche eingangsseitig mit einer Saugleitung in Verbindung steht, über die der zu untersuchende Prozeßstoff angesaugt werden kann. Ausgangsseitig steht die Pumpe über eine Druckleitung mit einem ersten Eingang eines Ventils in Verbindung, über das ein (schlauchförmiger) Probenbehälter entweder mit der Druckleitung oder mit einer Druckgasquelle verbindbar ist. Das andere Ende des schlauchförmigen Probenbehälters ist über ein weiteres Ventil entweder mit der Probenentnahmestelle oder aber mit dem Einfüllabschnitt verbindbar. Im Betrieb läßt man Prozeßflüssigkeit durch den schlauchförmigen Probenbehälter strömen, so daß dieser ständig mit einer den aktuellen Verhältnissen entsprechenden Proben gefüllt ist. Jedesmal, wenn eine Probe entnommen und nachfolgend deren Polyelektrolytgehalt bestimmt werden soll, werden die Ventile umgeschaltet, so daß der Inhalt des Probenbehälters von Druckgas beaufschlagt und in den Einfüllabschnitt gedrückt wird. Dadurch kann eine besonders hohe Reproduzierbarkeit der Probenentnahmemenge gesichert werden, wobei gleichzeitig die Gefahr von Verunreinigungen der Probe vermieden wird.

Weitere erfinderische Merkmale ergeben sich aus den Unteranansprüchen. Nachfolgend werden in der Beschreibung bevorzugte Ausführungsformen der Erfindung anhand von Abbildungen näher erläutert. Hierbei zeigen:
- Fig. 1: einen schematisierten Längsschnitt durch eine Ausführungsform der erfindungsgemäßen Vorrichtung; und
- Fig. 2: eine Schaltbild-Darstellung einer Ausführungsform der zur Erfindung gehörigen Probenentnahmeeinrichtung.

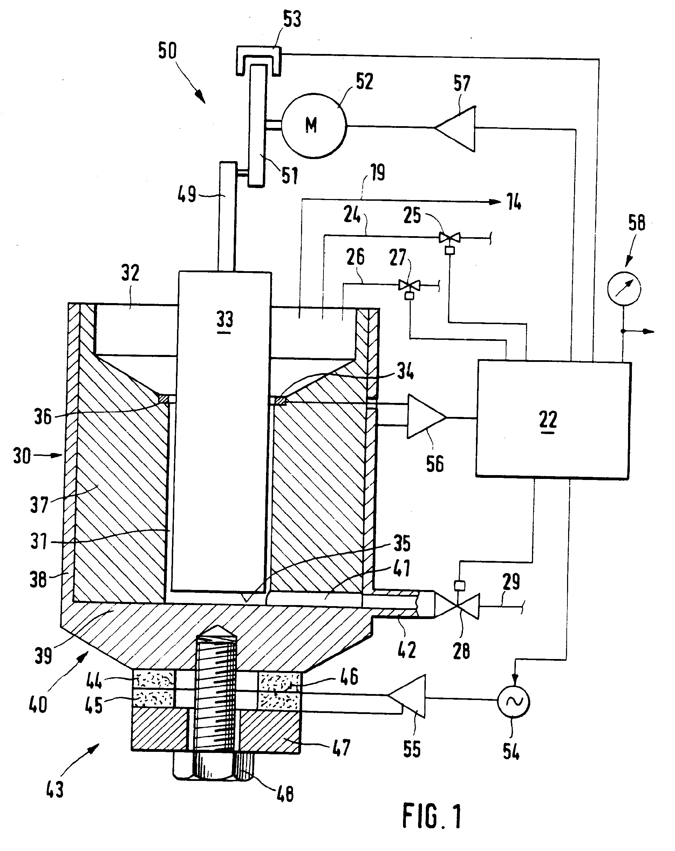

Bei der in Fig. 1 im Teil-Längsschnitt gezeigten Ausführungsform der Erfindung ist ein Probengefäß 30 vorgesehen, welches einen inneren Isolierblock 37 und ein äußeres Metallteil, bestehend aus einem Außenmantel 38 und einem Boden 39 aufweist. Der Isolierblock 37 weist eine im wesentlichen mittige Bohrung auf, welche einen zylindrischen Abschnitt 31 bildet, der nach oben in einen ebenfalls zylindrischen Einfüllabschnitt 32 größeren Durchmessers übergeht. An seinem oberen Ende ist im zylindrischen Abschnitt 31 eine Ringkerbe 36 vorgesehen, in welcher eine ringförmige erste Elektrode 34 aus einem korrosionsfesten Metall fest angebracht ist. Der Boden 39 schließt den zylindrischen Abschnitt 31 an seinem unteren Ende ab und bildet an dieser Stelle eine zweite Elektrode 35.

Die Elektroden 34 und 35 sind mit den Eingängen eines Verstärkers 56 verbunden, dessen Ausgang in einen Eingang einer Steuerung 22 geführt ist.

Der Boden 39 geht (einstückig) in einen Hornabschnitt 40 über, auf dessen Ende zwei übereinander gestapelte ringförmige Piezoschwinger mittels eines Schraubbolzens 48 und eines zwischengesetzten Gegenrings 47 gepreßt sind. Zwischen den Piezoringen 44, 45 ist eine Elektrode 46 angeordnet. Durch diese Anordnung sind die äußeren Ringflächen der beiden Piezoringe 44, 45 elektrisch leitend (über den Schraubbolzen 48) verbunden, so daß die beiden Piezoringe 44, 45 elektrisch parallel und mechanisch in Reihe geschaltet sind. Zur elektrischen Ansteuerung, die über die Elektrode 46 und die Metallteile 39/40, 47 und 48 erfolgt, ist ein Ultraschallgenerator 54 mit nachgeschaltetem Treiberverstärker 55 vorgesehen. Vorzugsweise ist die ganze Anordnung elektrisch derart rückgekoppelt, daß die Einstellung der Schwingfrequenz selbsttätig auf einen optimalen Wert erfolgt, der alle elektrischen und mechanischen Komponenten mit erfaßt.

In der unteren Endfläche des Isolierblocks 37 ist eine Kerbe 41 radial nach außen gerichtet angebracht, so daß eine Rohrleitung mit straßentunnelartigem Querschnitt entsteht, die einerseits von den Kerbenwänden, andererseits vom Boden 39 gebildet wird. An der Stelle, an welcher die Kerbe 41 auf den Außenmantel 38 trifft, ist in diesem eine Bohrung angebracht, welche in einen Rohrstutzen 42 übergeht. Der Rohrstutzen 42 ist über eine Leitung mit einem Magnetventil 28 verbunden, über welches der Rohrstutzen 42 mit einer Ablaßleitung 29 verbindbar ist. Das Magnetventil 28 ist über eine Steuerleitung mit der Steuerung 22 verbunden.

Im zylindrischen Abschnitt 31 steckt ein Kolben 33 aus isolierendem Material, der so dimensioniert ist, daß ein sehr enger Spalt (wenige Zehntel Millimeter) zwischen der Kolbenaußenfläche und dem zylindrischen Abschnitt 31 des Isolierkörpers 37 gebildet ist. Der Kolben 33 weist eine plane Endfläche auf und ist an der gegenüberliegenden Seite über einen Schaft 49 mit einer Kurbel 51 verbunden, die über einen Elektromotor 52 drehbar ist, wobei an der Kurbel 51 ein Drehwinkelgeber 53 angebracht ist, über welchen der Drehwinkel der Kurbel 51 und damit die Hubhöhe des Kolbens 33 abtastbar und der Steuerung 22 zuführbar sind. Der Elektromotor 52 ist über einen Treiberverstärker 57 von der Steuerung 22 ansteuerbar, so daß der so gebildete Antrieb 50 für den Kolben 33 sehr exakt gesteuert werden kann.

In den Einfüllabschnitt 32 münden eine Spülleitung 26, die über ein von der Steuerung 22 ansteuerbares Magnetventil 27 mit einem Behälter mit Spülflüssigkeit (destilliertes Wasser) in Verbindung bringbar ist. Weiterhin mündet eine Titerleitung 24 in den Einfüllabschnitt 32, welche über ein von der Steuerung 22 ansteuerbares Magnetventil 25 mit einem Vorratsbehälter für den Titer in Verbindung bringbar ist. Schließlich mündet eine Leitung 19 in den Einfüllabschnitt 32, über welche eine Probenflüssigkeit in den Einfüllabschnitt 32 einfüllbar ist. Auch dieses Einfüllen einer Probe geschieht unter Kontrolle durch die Steuerung 22.

Die Arbeitsweise der so aufgebauten Vorrichtung wird im folgenden beschrieben.

Es wird eine definierte Menge einer Probe durch die Leitung 19 in den Einfüllabschnitt 32 eingeführt. Der Kolben 33 wird über den Antrieb 50 auf- und abbewegt und das so entstehende Strömungspotential wird über die Elektrode 34, 35 und den Verstärker 56 der Steuerung 22 zugeführt, die den Meßwert verarbeitet und über eine Meßeinrichtung 58 anzeigt bzw. protokolliert oder anderen, nicht gezeigten Anlagenteilen als Steuer- bzw. Regelgröße zur Verfügung stellt. Gleichzeitig wird über die Leitung 24 titriert.

Nach Durchführung der Messung wird das Ventil 28 bei jeder Abwärtsbewegung des Kolbens 33 geöffnet und bei jeder Aufwärtsbewegung geschlossen. Dadurch wird die gesamte, in der Vorrichtung enthaltene Flüssigkeit in die Leitung 29 gepumpt und kann verworfen werden. Nach einer hinreichenden Pumpdauer, wenn also keine wesentlichen Probenmengen mehr in der Vorrichtung enthalten sind, wird das Spülventil 27 geöffnet, so daß Spülflüssigkeit über die Leitung 26 in den Einfüllabschnitt 32 eingeführt werden kann. Während dessen wird der Kolben 33 nach wie vor auf- und abbewegt und das Ventil 28 synchron hierzu wie oben beschrieben geöffnet und geschlossen. Gleichzeitig wird der Ultraschallgenerator 54 von der Steuerung 22 eingeschaltet, so daß in die Spülflüssigkeit Ultraschallschwingungen eingeleitet werden. Durch die Kavitationswirkung der Ultraschallschwingungen in der Flüssigkeit und den Durchspülvorgang unter gleichzeitiger Bewegung des Kolbens 33 findet eine gründliche Reinigung der mit der Probe in Berührung kommenden Teile statt. Sogar der Kanal 41 kann gründlich gereinigt werden, da der vom Ultraschallschwinger 43 in Schwingung versetzte Boden 39 ein Teil seiner Umfangswand bildet.

Wenn der Spülvorgang eine hinreichende Zeit angedauert hat, wird der Zufluß von Spülflüssigkeit durch Schließen des Ventils 27 gestoppt, woraufhin der Rest an Spülflüssigkeit durch Bewegung des Kolbens 33 abgepumpt wird. Nun kann erneut eine Probe eingefüllt werden.

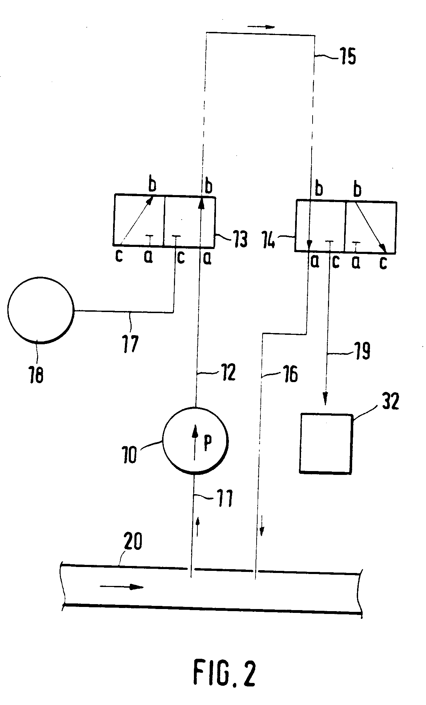

Zur Entnahme einer Probe aus einer Prozeßleitung 20 (oder einem Prozeßgefäß) wird vorteilhafterweise eine Vorrichtung verwendet, die in Fig. 2 schematisiert dargestellt ist. Diese Vorrichtung umfaßt eine Saugleitung 11, welche an die Prozeßleitung 20 bzw. an ein Prozeßgefäß angeschlossen ist und mit der Eingangsseite einer Pumpe 10 in Verbindung steht. Auf der Druckseite steht die Pumpe 10 über eine Druckleitung 12 mit einem Eingang a eines ersten Magnetventils 13 in Verbindung, dessen Ausgang b an ein Ende eines Probenschlauches 15 angeschlossen ist. Der Probenschlauch 15 steht mit seinem anderen Ende mit einem Eingang b eines zweiten Magnetventils 14 in Verbindung, das mit seinem einen Ausgang a über eine Abführleitung 16 wiederum mit der Prozeßleitung 20 (oder einem Prozeßgefäß) kommuniziert. In der in Fig. 2 gezeigten Stellung der Magnetventile 13, 14 kann somit ständig aus der Prozeßleitung 20 entnommene Flüssigkeit durch den Probenschlauch 15 hindurchgepumpt werden, so daß der Inhalt des Probenschlauches 15 mit dem momentanen Inhalt der Prozeßleitung 20 übereinstimmt.

Ein zweiter Eingang c des ersten Ventils 13 ist über eine Druckgasleitung 17 mit einer Druckgasquelle 18 verbunden. Ein zweiter Ausgang c des zweiten Ventils 14 ist mit der Entnahmeleitung 19 verbunden, die (wie oben beschrieben) in den Einfüllabschnitt 32 mündet.

Werden nun die Magnetventil 13 und 14 umgeschaltet (in die in Fig. 2 äußeren Stellungen), so wird der Inhalt des Probenschlauches 15 sowie Rest-Inhalte der Ventile 13 und 14 vom Druckgas aus der Druckgasquelle 18 durch die Entnahmeleitung 19 in den Einfüllabschnitt 32 gedrückt, wobei man das Gas vorzugsweise so lange strömen läßt, bis auch Rest-Tröpfchen in den Einfüllabschnitt 32 gefördert wurden. Durch diese Art der Probenentnahme gelingt eine ungewöhnlich exakte reproduzierbare Dosierung auf einfachste Weise, wobei gleichzeitig die Probe in ihrer Zusammensetzung nicht verändert wird. Darüber hinaus ist die Probenentnahmevorrichtung auch gegen ansonsten nur schwer zu verarbeitende Flüssigkeiten mit möglicherweise abrasiven Feststoffen unempfindlich, da alle Teile einerseits mit Prozeßflüssigkeit, andererseits mit Druckluft jeweils im Überfluß "gespült" werden. Dies ist gerade bei der Abwasserüberwachung besonders wichtig und ergänzt sich mit der besonders wirksamen Reinigung des in Fig. 1 gezeigten Vorrichtungsteils.

Zur Überwachung des Oberflächenzustands der Elektroden 34 und 35 kann bei im wesentlichen gleichbleibender Flüssigkeit das sich einstellende Anfangspotential herangezogen werden. Bei einer anderen Ausführungsform der Erfindung wird in regelmäßigen Abständen eine gleichbleibende Norm-Flüssigkeit anstelle einer Probe in den Einfüllabschnitt 32 eingefüllt, so daß das Anfangspotential einen exakten Meßwert zur Beurteilung des Oberflächenzustandes der Elektroden 34 und 35 liefert. Sobald das Anfangspotential unter einen Grenzwert sinkt, steuert die Steuerung 22 eine Warneinrichtung an, so daß die Elektroden gewartet werden können.

### Bezugszeichenliste

- 10: Pumpe
- 11: Saugleitung
- 12: Druckleitung
- 13: erstes Ventil
- 14: zweites Ventil
- 15: Probenschlauch
- 16: Abführleitung
- 17: Druckgasleitung
- 18: Druckgasquelle
- 19: Entnahmeleitung
- 20: Prozeßleitung
- 22: Steuerung
- 24: Titerleitung
- 25: Titerventil
- 26: Spülleitung
- 27: Spülventil
- 28: Ablaßventil
- 29: Ablaßleitung
- 30: Probengefäß
- 31: zylindrischer Abschnitt
- 32: Einfüllabschnitt
- 33: Kolben
- 34: erste Elektrode
- 35: zweite Elektrode
- 36: Ringkerbe
- 37: Isolierblock
- 38: Außenmantel
- 39: Boden
- 40: Hornabschnitt
- 41: Kerbe
- 42: Rohrstutzen
- 43: Ultraschallschwinger
- 44: Piezoring
- 45: Piezoring
- 46: Elektrode
- 47: Gegenring
- 48: Schraubbolzen
- 49: Schaft
- 50: Antrieb
- 51: Kurbel
- 52: Motor
- 53: Drehwinkelgeber
- 54: Ultraschallgenerator
- 55: Ultraschalltreiber
- 56: Ladungsverstärker
- 57: Motortreiber
- 58: Meßeinrichtung

## Patentansprüche

1. Vorrichtung zur automatisierten Polyelektrolytbestimmung, insbesondere in Prozeßstoffen, umfassend
ein Probengefäß (30) mit einem elektrisch isolierenden zylindrischen Innenabschnitt (31), einem darüberliegenden, anschließenden Einfüllabschnitt (32) größeren Durchmessers,
Elektroden (34, 35) im wesentlichen im Bereich der Enden des zylindrischen Innenabschnittes (31),
einem elektrisch isolierenden Kolben (33), der mit einem definierten geringen Spiel im zylindrischen Innenabschnitt motorisch auf- und abbewegbar ist, und
mit Einrichtungen (56) zur Messung einer Ladungsverschiebung zwischen den Elektroden (34, 35),
**gekennzeichnet** durch
eine Abflußleitung (29, 41, 42), die am Boden des zylindrischen Abschnitts (31) mündet,
eine Spülleitung (26) mit Ventil (27) zum gesteuerten Zuführen von Spülflüssigkeit in den Einfüllabschnitt (32) und durch
eine Steuerung (22), die mit Einrichtungen (50) zum Antreiben des Kolbens (33), einem Ventil (28) in der Abflußleitung (29, 41, 42) und dem Spülventil (27) verbunden und derart ausgebildet ist, daß nach jeder Durchführung einer Polyelektrolytbestimmung der untersuchte Stoff durch die Abflußleitung (29, 41, 42) abgeführt, Spülflüssigkeit zugeführt und unter Bewegung des Kolbens (33) durch die Abflußleitung (29, 41, 42) abgeführt wird.

2. Vorrichtung nach Anspruch 1,
dadurch gekennzeichnet,
daß die Steuerung (22) derart ausgebildet ist, daß beim Spülen das Abflußventil (28) während einer Aufwärtsbewegung des Kolbens (33) geschlossen und während einer Abwärtsbewegung des Kolbens (33) geöffnet ist.

3. Vorrichtung nach einem der vorhergehenden Ansprüche,
dadurch gekennzeichnet,
daß der Antrieb (50) einen Positionsfühler (53) zum Abtasten der Position des Kolbens (33) umfaßt.

4. Vorrichtung nach einem der vorhergehenden Ansprüche,
gekennzeichnet durch einen Ultraschallschwinger (43), der mit dem Probengefäß (30) mechanisch gekoppelt und während eines Spülvorganges derart ansteuerbar ist, daß Ultraschallschwingungen in die Spülflüssigkeit einkoppelbar sind.

5. Vorrichtung nach Anspruch 4,
dadurch gekennzeichnet,
daß mindestens der Boden (39) des zylindrischen Abschnitts (31) einen Metallkörper umfaßt, an den der Ultraschallschwinger (43) gekoppelt ist.

6. Vorrichtung nach Anspruch 5,
dadurch gekennzeichnet,
daß der Boden (39) über einen Hornabschnitt (40) zur Impedanzanpassung an den Ultraschallschwinger (43) gekoppelt ist.

7. Vorrichtung nach Anspruch 6,
dadurch gekennzeichnet,
daß der Boden (39) einstückig mit dem Hornabschnitt (40) ausgebildet ist.

8. Vorrichtung nach einem der vorhergehenden Ansprüche,
dadurch gekennzeichnet,
daß das Probengefäß (30) ein Außengefäß (38, 39) aus Metall mit einem im wesentlichen zylindrischen Innenraum und einen in diesen eingepaßten Isolierkörper (37) umfaßt, in den der zylindrische Abschnitt (31) und der Einfüllabschnitt (32) als ineinander übergehende abgestufte und offene Bohrungen eingearbeitet sind.

9. Vorrichtung nach Anspruch 8,
dadurch gekennzeichnet,
daß der zylindrische Innenraum des Außengefäßes (38, 39) einen zu seiner Zylinderlängsachse senkrechte, den Boden (39) bildende und plane Abflußfläche aufweist, in deren Höhe ein äußerer Abschnitt der Abflußleitung (42) mündet, und daß der Isolierkörper (37) in einer, dem Boden (39) zugewandten Endfläche eine, durch den Boden (39) abgeschlossene, in radialer Richtung zum zylindrischen Abschnitt (31) verlaufende Kerbe aufweist, welche einen inneren Abschnitt (41) der Abflußleitung bildet.

10. Vorrichtung nach einem der Ansprüche 8 oder 9,
dadurch gekennzeichnet,
daß der Isolierkörper (37) aus Polytetrafluorethylen gefertigt ist.

11. Vorrichtung nach einem der Ansprüche 8 bis 10,
dadurch gekennzeichnet,
daß das Probengefäß (30) aus V₂A-Stahl gefertigt ist.

12. Vorrichtung nach einem der Ansprüche 5 bis 11,
dadurch gekennzeichnet,
daß der Boden (39) die eine (35) der Elektroden (34, 35) bildet.

13. Vorrichtung nach einem der vorhergehenden Ansprüche,
gekennzeichnet durch eine Einrichtung zur Entnahme von Proben der flüssigen Prozeßstoffe, umfassend Pumpeinrichtungen (10), die eingangsseitig mit einer Saugleitung (11), welcher der Prozeßstoff zuführbar ist, und ausgangsseitig mit einer Druckleitung (12) in Verbindung stehen, von der Steuerung (22) steuerbare erste Ventileinrichtungen (13), über welche die Druckleitung (12) mit einem Probenbehälter 15 in Verbindung bringbar ist, von der Steuerung (22) steuerbare zweite Ventileinrichtungen (14), über welche der Probenbehälter (15) mit einer Abführleitung (16) verbindbar ist, um den von den Pumpeinrichtungen (10) dem Prozeß entnommenen und durch den Probenbehälter (15) geförderten Prozeßstoff wieder dem Prozeß zuzuführen, und Druckgaseinrichtungen (17, 18), um den Inhalt des Probenbehälters (15) mittels direkter Druckbeaufschlagung durch ein Gas durch eine Entnahmeleitung (19) im Einführabschnitt (32) zuzuführen.

14. Vorrichtung nach Anspruch 13,
dadurch gekennzeichnet,
daß der Probenbehälter (15) als Leitung ausgebildet ist, durch die der Prozeßstoff hindurchförderbar ist.

15. Vorrichtung nach einem der Ansprüche 13 oder 14,
dadurch gekennzeichnet,
daß die Ventileinrichtungen (14, 15) jeweils ein Dreiwege-Ventil umfassen, die in einer ersten Stellung die Druckleitung (12) mit dem Probenbehälter (15) und diesen mit der Abführleitung (16) und in einer zweiten Stellung die Druckgaseinrichtungen (17, 18) mit dem Probenbehälter (15) und diesen mit der Entnahmeleitung (19) verbinden.

16. Vorrichtung nach einem der Ansprüche 13 bis 15,
dadurch gekennzeichnet,
daß die Druckgaseinrichtungen eine Druckgasquelle (18) zum Liefern eines unter Druck stehenden Inertgases umfassen.

## Claims

1. Device for the automatic determination of polyelectrolyte, particularly in process substances, comprising a sample vessel (30) with an electrically insulating cylindrical internal section (31), an adjoining filling section (32) of larger diameter located above,
electrodes (34, 35) essentially in the area of the ends of the cylindrical internal section (31),
an electrically insulating piston (33), which can be moved upwards and downwards by motor power with a defined small clearance in the cylindrical internal section, and with devices (56) for measuring a charge transfer between the electrodes (34, 35),
characterized by an outlet line (29, 41, 42) which opens at the bottom of the cylindrical section (31), a flushing line (26) with valve (27) for the controlled supply of flushing liquid into the filling section (32) and by a control (22) which is connected to devices (50) for driving the piston (33), a valve (28) in the outlet line (29, 41, 42) and the flush valve (27) and is designed in such a way that after each performance of a polyelectrolyte determination the substance examined is discharged through the outlet line (29, 41, 42), flushing liquid is supplied and discharged through the outlet line (29, 41, 42) under movement of the piston (33).

2. Device according to Claim 1,
characterized in that
the control (22) is designed in such a way that in the course of flushing the outlet valve (28) is closed in the course of an upwards movement of the piston (33) and is opened in the course of a downwards movement of the piston (33).

3. Device according to one of the preceding Claims,
characterized in that
the drive (50) comprises a position sensor (53) for scanning the position of the piston (33).

4. Device according to one of the preceding Claims,
characterized by an ultrasonic oscillator (43) which is mechanically coupled to the sample vessel (30) and can be controlled in the course of flushing process in such a way that ultrasonic oscillations can be coupled into the flushing liquid.

5. Device according to Claim 4,
characterized in that
at least the bottom (39) of the cylindrical section (31) comprises a metal body to which the ultrasonic oscillator (43) is coupled.

6. Device according to Claim 5,
characterized in that
the bottom (39) is coupled to the ultrasonic oscillator (43) via a horn section (40) for impedance matching.

7. Device according to Claim 6,
characterized in that
the bottom (39) is designed to be a single piece with the horn section (40).

8. Device according to one of the preceding Claims,
characterized in that
the sample vessel (30) comprises an external vessel (38, 39) of metal with an essentially cylindrical internal chamber and an insulating body (37) fitted into this, in which the cylindrical section (31) and the filling section (32) are incorporated as graduated and open holes blending into each other.

9. Device according to Claim 8,
characterized in that
the cylindrical internal chamber of the external vessel (38, 39) has a flat outlet surface forming the bottom (39) and perpendicular to its cylinder longitudinal axis, at whose level an external section of the outlet line (42) opens, and that in an end surface facing the bottom (39) the insulating body (37) has a notch closed off by the bottom (39) and running in the radial direction to the cylindrical section (31), which notch forms an internal section (41) of the outlet line.

10. Device according to one of Claims 8 or 9,
characterized in that
the insulating body (37) is made of polytetrafluoroethylene.

11. Device according to one of Claims 8 to 10,
characterized in that
the sample vessel (30) is made of V₂A steel.

12. Device according to one of Claims 5 to 11,
characterized in that
the bottom (39) forms the one (35) of the electrodes (34, 35).

13. Device according to one of the preceding Claims,
characterized by a device for taking samples of the liquid process substances, comprising pump devices (10) which are connected on the inlet side to a suction line (11) to which the process substance can be fed and on the outlet side to a pressure line (12), first valve devices (13) controllable by the control (22) and via which the pressure line (12) can be brought into connection with a sample container (15), second valve devices (14) controllable by the control (22) and via which the sample container (15) can be connected to an outlet line (16) in order to return to the process the process substance taken from the process by the pump devices (10) and conveyed through the sample container (15), and compressed gas devices (17, 18) in order to convey the contents of the sample container (15) by means of direct pressure application by means of a gas through a take-off line (19) in the entry section (32).

14. Device according to Claim 13,
characterized in that
the sample container (15) is designed as a line through which the process substance can be conveyed.

15. Device according to one of Claims 13 or 14,
characterized in that
the valve devices (14, 15) comprise a three-way valve in each case which in a first position connect the pressure line (12) to the sample container (15) and this to the outlet line (16) and in a second position connect the compressed gas devices (17, 18) to the sample container (15) and this to the take-off line (19).

16. Device according to one of Claims 13 to 15,
characterized in that
the compressed gas devices comprise a compressed gas source (18) for supplying a pressurized inert gas.

## Revendications

1. Dispositif de détermination automatisée de polyélectrolytes, dans des substances industrielles notamment, comportant un vase d'échantillonnage (30) muni d'une section interne cylindrique (31), isolée électriquement, d'une section de remplissage (32) sus-jacente, de diamètre supérieur, d'électrodes (34, 35), essentiellement disposées dans la zone des extrémités cylindriques de la section interne (31), d'un piston (33) isolé électriquement, qu'un moteur permet de lever et d'abaisser dans la section cylindrique interne, avec un léger jeu défini, et de dispositifs (56) pour la mesure d'un transfert de charge entre les électrodes (34, 35), caractérisé par une conduite de décharge (29, 41, 42), qui débouche sur le fond de la section cylindrique (31), par une conduite de rinçage (26) avec une soupape (27), pour une arrivée contrôlée du liquide de rinçage dans la section de remplissage (32), et par une commande (22), reliée à des dispositifs (50) pour l'entraînement du piston (33), à une soupape (28) montée dans la conduite de décharge (29, 41, 42), et à la soupape de rinçage (27), cette commande étant réalisée de sorte que, après chaque détermination de polyélectrolytes, la substance analysée est évacuée par la conduite de décharge (29, 41, 42), du liquide de rinçage est amené et évacué par la conduite de décharge (29, 41, 42), sous l'effet du déplacement du piston (33).

2. Dispositif suivant la revendication 1, caractérisé en ce que la commande (22) a une réalisation telle que, lors du rinçage, la soupape de décharge (28) est fermée pendant un déplacement vers le haut du piston (33), et ouverte pendant un déplacement vers le bas du piston (33).

3. Dispositif suivant l'une quelconque des revendications précédentes, caractérisé en ce que la commande (50) comporte un capteur de position (53) pour détecter la position du piston (33).

4. Dispositif suivant l'une quelconque des revendications précédentes, caractérisé par un oscillateur ultrasonore (43), qui est couplé mécaniquement au vase d'échantillonnage (30) et peut être attaqué, pendant une opération de rinçage, de sorte que des vibrations ultrasonores peuvent être transmises au liquide de rinçage.

5. Dispositif suivant la revendication 4, caractérisé en ce que le fond (39), au moins, de la section cylindrique (31), comporte un corps métallique, auquel est couplé l'oscillateur ultrasonore (43).

6. Dispositif suivant la revendication 5, caractérisé en ce que le fond (39) est couplé à l'oscillateur ultrasonore (43), par l'intermédiaire d'une section en cornet (40), pour l'adaptation d'impédance.

7. Dispositif suivant la revendication 6, caractérisé en ce que le fond (39) est réalisé d'une seule pièce avec la section en cornet (40).

8. Dispositif suivant l'une quelconque des revendications précédentes, caractérisé en ce que le vase d'échantillonnage (30) comporte un vase externe (38, 39) en métal, avec un compartiment interne essentiellement cylindrique, et un corps isolant (37) encastré dans ce dernier, dans lequel sont pratiquées la section cylindrique (31) et la section de remplissage (32) sous forme de trous ouverts et étagés, situés dans le prolongement l'un de l'autre.

9. Dispositif suivant la revendication 8, caractérisé en ce que le compartiment interne cylindrique du vase externe (38, 39) présente une surface de décharge plane, perpendiculaire à son axe longitudinal cylindrique, formant le fond (39), et au niveau de laquelle débouche une section externe de la conduite de décharge (42), et en ce que le corps isolant (37) présente, dans une surface extrême tournée vers le fond (39), une rainure fermée par le fond (39), située dans le sens radial par rapport à la section cylindrique (31), et qui forme une section interne (41) de la conduite de décharge.

10. Dispositif suivant l'une quelconque des revendications 8 et 9, caractérisé en ce que le corps isolant (37) est réalisé en polytétrafluoroéthylène.

11. Dispositif suivant l'une quelconque des revendications 8 à 10, caractérisé en ce que le vase d'échantillonnage (30) est réalisé en acier V₂A.

12. Dispositif suivant l'une quelconque des revendications 5 à 11, caractérisé en ce que le fond (39) constitue l'une (35) des électrodes (34, 35).

13. Dispositif suivant l'une quelconque des revendications précédentes, caractérisé par un appareillage pour le prélèvement d'échantillons de substances industrielles liquides, comportant des systèmes de pompage (10), reliés côté entrée à une conduite d'aspiration (11), qui peut être alimentée en substance industrielle, et côté sortie à une conduite de refoulement (12), des premiers systèmes de vanne (13) pouvant être pilotés par la commande (22), et par l'intermédiaire desquels la conduite de refoulement (12) peut être reliée à un récipient d'échantillonnage (15), des seconds systèmes de vanne (14) pouvant être pilotés par la commande (22), et par l'intermédiaire desquels le récipient d'échantillonnage (15) peut être relié à une conduite d'évacuation (16), pour renvoyer dans le procédé la substance industrielle, soutirée du procédé par les systèmes de pompage (10) et véhiculée par le récipient d'échantillonnage (15), et des systèmes de gaz sous pression (17, 18), pour envoyer dans la section de remplissage (32), au travers d'une conduite de soutirage (19), le contenu du récipient d'échantillonnage (15) par une alimentation directe en gaz sous pression.

14. Dispositif suivant la revendication 13, caractérisé en ce que le récipient d'échantillonnage (15) est réalisé sous forme de conduite, au travers de Laquelle peut être véhiculée la substance industrielle.

15. Dispositif suivant l'une quelconque des revendications 13 et 14, caractérisé en ce que les système de vanne (14, 15) comportent respectivement une vanne à trois voies, qui relie dans une première position la conduite de refoulement (12) au récipient d'échantillonnage (15), et ce dernier à la conduite d'évacuation (16), et dans une seconde position les systèmes de gaz sous pression (17, 18) au récipient d'échantillonnage (15), et ce dernier à la conduite de soutirage (19).

16. Dispositif suivant l'une quelconque des revendications 13 à 15, caractérisé en ce que les systèmes de gaz sous pression comportent une source de gaz sous pression (18), pour fournir un gaz inerte sous pression.
